# EUROPEAN PATENT APPLICATION

(11) **EP 4 509 510 A1**
(43) Date of publication of application: **19.02.2025**
(21) Application number: 23788540.5
(22) Date of filing: 07.04.2023
(51) Int. Cl.: C07D 493/04, A61K 31/352

(54) **SYNTHESIS METHOD FOR 3-PHENYL-2,3,4,8,9,10-HEXAHYDROPYRANO[2,3-F]CHROMENE DERIVATIVE**

(30) Priority: 12.04.2022 KR 20220045022
(71) Applicant: Glaceum, Inc., Gyeonggi-do 16675 (KR)
(72) Inventor: YOO, Sang Ku, Suwon-si Gyeonggi-do 16224 (KR); LIM, Jeong Ho, Yongin-si Gyeonggi-do 16989 (KR); KANG, Ku Suk, Yongin-si Gyeonggi-do 17095 (KR); KIM, Jin Young, Pyeongtaek-si Gyeonggi-do 18016 (KR); LEE, Jung Woo, Anyang-si Gyeonggi-do 14069 (KR); YUN, Jae Jeong, Suwon-si Gyeonggi-do 16675 (KR)
(74) Representative: Cabinet Nony
(86) International application number: PCT/KR2023/004744
(87) International publication number: WO 2023/200189

(57) **Abstract**

The present invention relates to a method for synthesis of 3-phenyl-2,3,4,8,9,10-hexahydropyrano[2,3-f]chromene derivatives.

## Description

### Technical Field

This application claims the benefit of the filing date of Korean Patent Application No. 10-2022-0045022, filed with the Korean Intellectual Property Office on April 12, 2022, the entire contents of which are incorporated in the present invention.

The present invention relates to a method for synthesizing 3-phenyl-2,3,4,8,9,10-hexahydropyrano[2,3-f]chromene derivatives.

### Background Art

Patent Document 1 [Korean Patent No. 10-2015-0075030] discloses that pyranochromenylphenol derivatives are effective in preventing and treating various metabolic syndromes, including hyperlipidemia, fatty liver, glucose metabolism disorders, diabetes, and obesity. Patent Document 2 [Korean Patent No. 10-2019-0116100] is an invention relating to a method of synthesizing these pyranochromenylphenol derivatives according to the following Reaction Scheme in order to synthesize pyranochromenylphenol derivatives. That is, Patent Document 2 discloses a method capable of synthesizing Formula F by coupling Formula A and Formula B together to obtain Formula C, which is then cyclized to obtain Formula D, which is then reduced to obtain Formula E, which is then subjected to reductive elimination. Formula F may then be synthesized into various derivatives disclosed in Patent Document 1 [Korean Patent No. 10-2015-0075030] through simple hydrogenation.

Here, since the process of producing Formula F from Formula E employs McMurry reagent [Ti(0) or Ti(1⁺)] prepared from Zn metal and TiCl₄, there is difficulty in controlling and refining metal impurities, and commercial mass production involves considerable effort and is difficult. Therefore, a synthetic method capable of mass production under simple and mild conditions is needed.

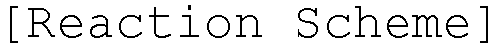

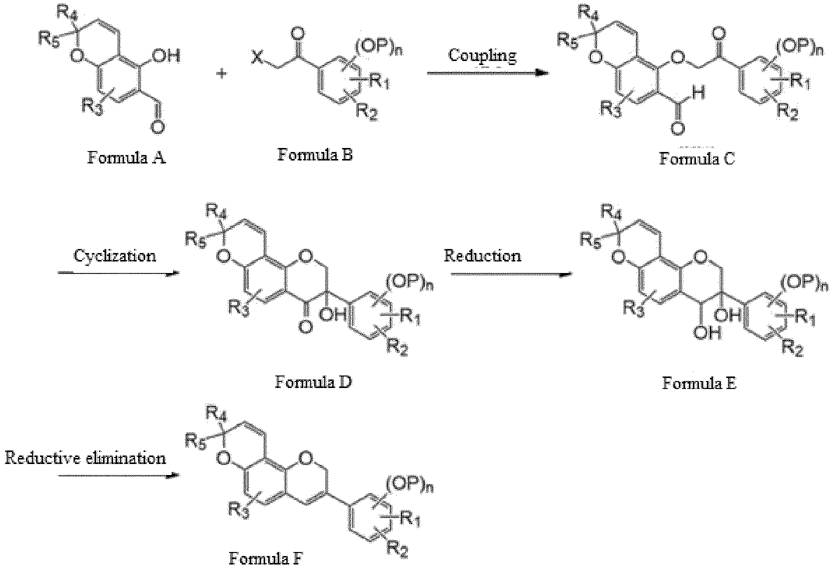

### DISCLOSURE

### Technical Problem

The present invention is intended to provide a synthetic method capable of easily preparing 3-phenyl-2,3,4,8,9,10-hexahydropyrano[2,3-f]chromene derivatives.

However, the problems to be solved by the present invention are not limited to the problems mentioned above, and other problems not mentioned above will be clearly understood by those skilled in the art from the following description.

### Technical Solution

One embodiment of the present invention provides a method for synthesizing 3-phenyl-2,3,4,8,9,10-hexahydropyrano[2,3-f]chromene derivatives.

### Advantageous Effects

Through a synthetic method according to one embodiment of the present invention, it is possible to effectively prepare 3-phenyl-2,3,4,8,9,10-hexahydropyrano[2,3-f]chromene derivatives.

In addition, the synthetic method according to one embodiment of the present invention may effectively prepare 3-phenyl-2,3,4,8,9,10-hexahydropyrano[2,3-f]chromene derivatives under simple and mild conditions.

In addition, the synthetic method according to one embodiment of the present invention may commercially massproduce 3-phenyl-2,3,4,8,9,10-hexahydropyrano[2,3-f]chromene derivatives.

In addition, the synthetic method according to one embodiment of the present invention has an advantage over conventional synthetic methods in that it may overcome the problem of difficulty in controlling and refining metal impurities.

However, the effects of the present invention are not limited to the effects described above, and may be expanded in various ways without departing from the spirit and scope of the present invention.

### Best Mode

Throughout the present specification, it is to be understood that when any part is referred to as "comprising" any component, it does not exclude other components, but may further comprise other components, unless otherwise specified.

Throughout the present specification, when any member is referred to as being "on" another member, it not only refers to a case where any member is in contact with another member, but also a case where a third member exists between the two members.

Hereinafter, the present invention will be described in more detail.

One embodiment of the present invention provides a method for synthesizing a 3-phenyl-2,3,4,8,9,10-hexahydropyrano[2,3-f]chromene derivative, comprising steps of: dehydrating a compound represented by the following Formula 1 in the following Reaction Scheme 1 to prepare a compound represented by the following Formula 2; and
reducing (hydrogenating) the compound represented by Formula 2 to prepare a compound represented by the following Formula 3:

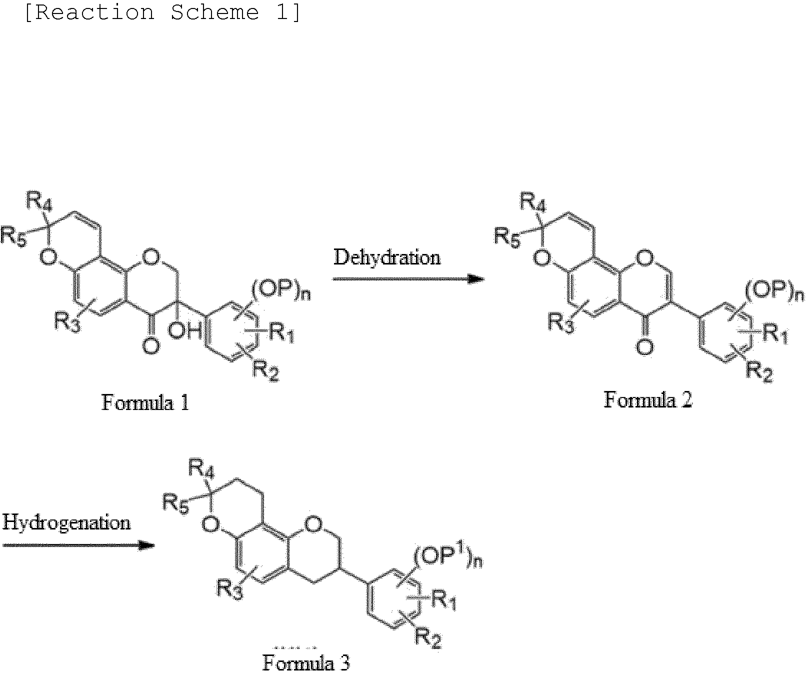

In Reaction Scheme 1,
R₁ and R₂ are each independently a hydrogen atom; a substituted or unsubstituted, linear or branched C₁-C₆ alkyl group; a halogen atom; a substituted or unsubstituted, linear or branched C₁-C₆ alkoxy group; a substituted or unsubstituted, linear or branched C₁-C₄ thioalkyl group; a substituted or unsubstituted C₆-C₁₂ aryl group; a substituted or unsubstituted allyloxy group; or a substituted or unsubstituted C₆-C₁₂ aryloxy group;
R₃ is a hydrogen atom, a C₁-C₂ alkyl group, a C₁-C₂ alkoxy group, or a halogen atom;
R₄ and R₅ are each independently a hydrogen atom or a C₁-C₆ alkyl group;
P represents a protecting group of a substituted or unsubstituted benzyl group, a substituted or unsubstituted allyl group, methanesulfonyl (MeSO₂), p-toluenesulfonyl (p-TsSO₂), or trimethylphenylsulfonyl;
P¹ is a hydrogen atom, a substituted or unsubstituted allyl group, methanesulfonyl (MeSO₂), p-toluenesulfonyl (p-TsSO₂), or trimethylphenylsulfonyl;
n is 1 to 3;
when OP is plural, OPs are the same or different; and
the substituent of each of the substituted alkyl group, the substituted alkoxy group, the substituted thioalkyl group, the substituted aryl group, the substituted allyloxy group, the substituted aryloxy group, the substituted benzyl group and the substituted allyl group is a benzyloxy group, a halogen atom, a linear or branched C₁-C₅ alkyl group, a linear or branched C₁-C₅ alkoxy group, a linear or branched C₁-C₃ thioalkyl group, or a nitro group.

When P in Reaction Scheme 1 above is a substituted or unsubstituted benzyl group, if the hydrogenation reaction is performed using Pd/C as a catalyst, the substituted or unsubstituted benzyl group may be removed, leaving hydrogen. In this case, therefore, P in Formula 2 may be a substituted or unsubstituted benzyl group, and P¹ in Formula 3 may be hydrogen. In addition, since a substituted or unsubstituted allyl group, MeSO₂, p-TsSO₂ or trimethylphenylsulfonyl does not react under hydrogenation reaction conditions, P in Formula 2 and P¹ in Formula 3 may be the same.

In the method for synthesizing 3-phenyl-2,3,4,8,9,10-hexahydropyrano[2,3-f]chromene derivatives according to one embodiment of the present invention, in Reaction Scheme 1 above,
R₁ and R₂ are each independently a hydrogen atom; a substituted or unsubstituted, linear or branched C₁-C₃ alkyl group; or a linear or branched C₁-C₃ alkoxy group;
R₃ is a hydrogen atom;
R₄ and R₅ are each independently a hydrogen atom or a C₁-C₃ alkyl group;
P represents a substituted or unsubstituted benzyl group or p-toluenesulfonyl (p-TsSO₂); and
P¹ is a hydrogen atom or p-toluenesulfonyl (p-TsSO₂).

In the method for synthesizing 3-phenyl-2,3,4,8,9,10-hexahydropyrano[2,3-f]chromene derivatives according to one embodiment of the present invention, in Reaction Scheme 1 above,
the compound represented by Formula 1 may be any one compound selected from the group consisting of the following compounds:
3-(2-(benzyloxy)-4-propoxyphenyl)-3-hydroxy-8,8-dimethyl-2,3-dihydropyrano[2,3-f]chromen-4(8H)-one;
3-(2-(benzyloxy)-4-ethoxyphenyl)-3-hydroxy-8,8-dimethyl-2,3-dihydropyrano[2,3-f]chromen-4(8H)-one;
3-(2-(benzyloxy)-4-propylphenyl)-3-hydroxy-8,8-dimethyl-2,3-dihydropyrano[2,3-f]chromen-4(8H)-one; and
3-(2-(p-tosyloxy)-4-ethoxyphenyl)-3-hydroxy-8,8-dimethyl-2,3-dihydropyrano[2,3-f]chromen-4(8H)-one.

In the method for synthesizing 3-phenyl-2,3,4,8,9,10-hexahydropyrano[2,3-f]chromene derivatives according to one embodiment of the present invention, in Reaction Scheme 1 above,
the compound represented by Formula 3 may be any one compound selected from the group consisting of the following compounds:
2-(8,8-dimethyl-2,3,4,8,9,10-hexahydropyrano[2,3-f]chromen-3-yl)-5-propoxyphenol;
2-(8,8-dimethyl-2,3,4,8,9,10-hexahydropyrano[2,3-f]chromen-3-yl)-5-ethoxyphenol;
2-(8,8-dimethyl-2,3,4,8,9,10-hexahydropyrano[2,3-f]chromen-3-yl)-5-propylphenol; and
1-(8,8-dimethyl-2,3,4,8,9,10-hexahydropyrano[2,3-f]chromen-3-yl)-2-(p-tosyloxy)-4-ethoxybenzene.

When dehydration of the Formula 1 in Reaction Scheme 1 proceeds, the hydroxyl group at position 3 of Formula 1 is removed, and a double bond is formed at positions 2 and 3. When hydrogenation of the Formula 2 in Reaction Scheme 1 proceeds, the double bond of the pyran is converted to a single bond, and the ketone is reduced, thereby producing the Formula 3.

The dehydration in Reaction Scheme 1 may be performed under acidic conditions. For example, the dehydration may be performed in an acid solution containing at least one of sulfuric acid, nitric acid, hydrochloric acid, phosphoric acid, and p-toluenesulfonic acid. The dehydration may be performed at a temperature of 40 to 60°C or below.

By performing the dehydration under the above conditions, it is possible to stably prepare the compound represented by Formula 2 from the compound represented by Formula 1 in Reaction Scheme 1, and increase the yield of the compound of Formula 2.

The reduction (hydrogenation) in Reaction Scheme 1 may be performed through a hydrogenation using Pd/C (palladium on carbon) as a catalyst to perform both reduction of the double bond and removal of the carbonyl group, thereby producing the compound represented by Formula 3. Here, the reduction may be performed at a temperature of 0 to 60°C. In addition, the reduction may be performed at a hydrogen pressure of 1 to 7 atm.

By performing the reduction under the above conditions, it is possible to stably prepare the compound represented by Formula 3 from the compound represented by Formula 2 in Reaction Scheme 1 and to increase the yield of the compound of Formula 3.

The purpose of the present invention is to provide an economical method that fundamentally solves the problem of Patent Document 2 (Korean Patent No. 10-2019-0116100) by directly preparing the Formula 3 through dehydration of the Formula 1 and hydrogenation of Formula 2 in Reaction Scheme 1.

Conventional synthetic methods, including Korean Patent Publication No. 10-2019-0116100, require three steps (reduction, reductive elimination, and hydrogenation) to synthesize the Formula 3 from the Formula 1, and encounter difficulty in the handling of the reagent (McMurry reagent) used. The present invention has great advantages in terms of commercialization because it possible to synthesize the Formula 3 through only two steps (dehydration and hydrogenation) and the handling of the reagents used or the reaction conditions are not difficult.

### Nomenclature:

### Formula 1:

3,3-Phenylhydroxy-2,3-dihydropyrano[2,3-f]chromen-4(8H)-one derivatives

### Formula 2:

3-Phenyl-pyrano[2,3-f]chromen-4(8H)-one derivatives

### Formula 3:

3-Phenyl-2,3,4,8,9,10-hexahydropyrano[2,3-f]chromene derivatives

One embodiment of the present invention provides a compound represented by the following Formula 2:

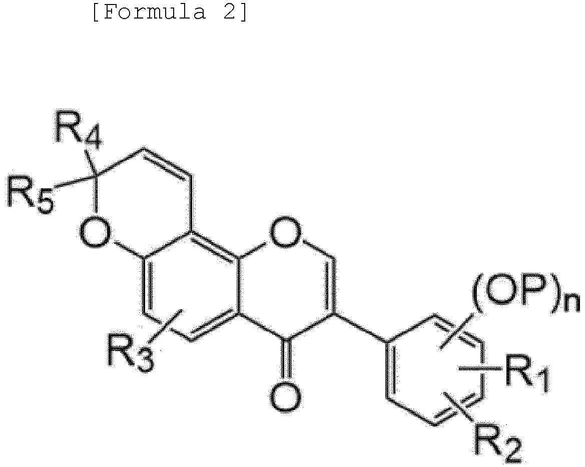

In Formula 2 above, R₁ to R₅ are the same as defined in Reaction Scheme 1 above.

Using the compound represented by Formula 2, the compound represented by Formula 3 may be easily prepared. In addition, the compound of Formula 3 may be prepared in a simple manner under milder conditions.

According to one embodiment of the present invention, the compound represented by Formula 1 in Reaction Scheme 1 may be prepared according to the following Reaction Scheme 2:

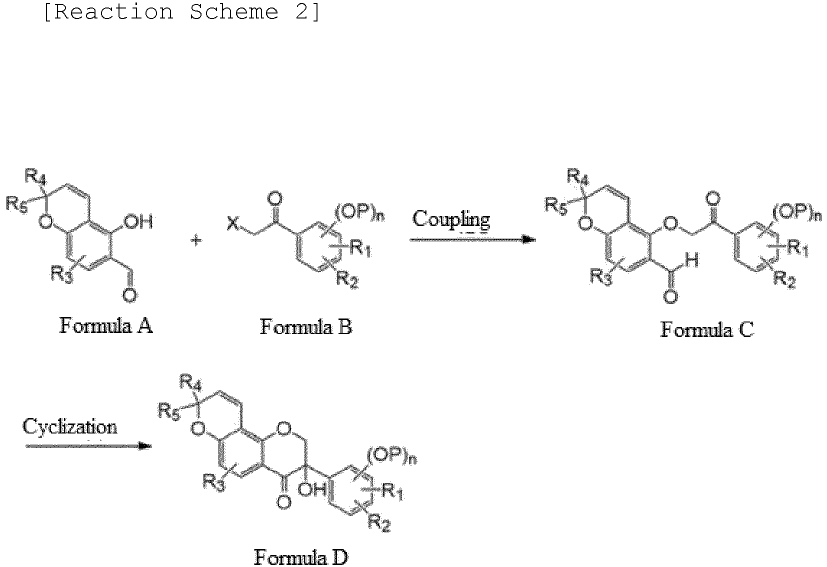

In Reaction Scheme 2 above, R₁ to R₅ are the same as defined in Reaction Scheme 1 above, and X may be a halogen. Formula D in Reaction Scheme 2 above may be Formula 1 in Reaction Scheme 1 above.

The coupling step in Reaction Scheme 2 above may be performed under basic conditions. The basic conditions may be formed by addition of at least one weakly basic compound selected from the group consisting of sodium carbonate (Na₂CO₃), lithium carbonate (Li₂CO₃), potassium carbonate (K₂CO₃), sodium bicarbonate (NaHCO₃), potassium bicarbonate (KHCO₃), triethylamine, and pyridine.

The cyclization in Reaction Scheme 2 may be performed to cyclization through benzoin condensation by adding a salt to the compound of Formula C, thereby obtaining the compound of Formula D. Specifically, the salt may be a thiazolium salt, a triazolium salt, or a 6,7-dihydro-5h-pyrrolo[2,1-c][1,2,4]triazolium salt.

### Mode for Invention

Hereinafter, the present invention will be described in detail by way of examples. However, the examples according to the present invention may be modified into various different forms, and the scope of the present invention is not interpreted as being limited to the examples described below. The examples of the present specification are provided to more completely explain the present invention to those skilled in the art.

### Examples

### Example 1: Preparation of 2-(8,8-dimethyl-2,3,4,8,9,10-hexahydropyrano[2,3-f]chromen-3-yl)-5-propoxyphenol

### 1-1. Synthesis of 3-(2-(benzyloxy)-4-propoxyphenyl)-8,8-dimethyl-pyrano[2,3-f]chromen-4(8H)-one

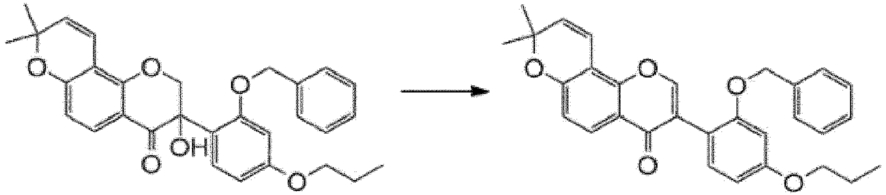

516 g of 95% sulfuric acid was added dropwise to 800 mL of THF at 0 to 20°C, and 50 g (0.1028 mol) of 3-(2-(benzyloxy)-4-propoxyphenyl)-3-hydroxy-8,8-dimethyl-2,3-dihydropyrano[2,3-f]chromen-4(8H)-one was added in portions to the solution at 20°C or lower. The mixture was warmed to 40 to 45°C and stirred for 2 hours. After completion of the reaction, the reaction solution was cooled to 20°C or below, 500 mL of purified water was added thereto, followed by stirring at 20 to 30°C for 1 hour. The reaction solution was filtered and the filtrate was washed with 250 mL of purified water at room temperature and then dried. 180 mL of methanol was added to 45.2 g of the dried product, followed by stirring at 55°C for 1 hour. The reaction solution was cooled to 25°C, stirred for 1 hour, filtered, and dried to obtain 42.8 g (yield 88.8%) of 3-(2-(benzyloxy)-4-propoxyphenyl)-8,8-dimethyl-pyrano[2,3-f]chromen-4(8H)-one.

¹H-NMR (CDCl₃) : 8.0648(d, 1H, J=8.76Hz), 7.9123(s, 1H), 7.3544~7.2182(m, 6H), 6.8447(d, 1H, J=8.76Hz), 6.7856(d, 1H, J=10.0Hz), 6.6035(s, 1H), 6.5644(d, 1H, J=8.52Hz), 5.6888(d, 1H, J=10.00Hz), 5.0573(s, 2H), 3.9161(t, 2H, J=6.52Hz), 1.7967(m, 2H), 1.4879(s, 6H), 1.0291(t, 3H, J=7.40Hz).

¹³C-NMR (CDCl₃) : 176.0145, 160.6824, 157.8164, 157.2211, 153.5956, 152.5656, 137.2333, 132.3996, 130.2681, 128.6029, 127.8214, 127.3682, 126.8727, 122.3239, 118.6639, 115.2862, 115.1127, 113.9942, 109.3931, 105.9793, 101.3679, 77.7617, 77.5482, 77.4322, 77.2300, 76.9125, 70.8135, 69.8359, 68.1466, 28.3101, 25.7900, 22.7301, 10.7101.

### 1-2. Synthesis of 2-(8,8-dimethyl-2,3,4,8,9,10-hexahydropyrano[2,3-f]chromen-3-yl)-5-propoxyphenol

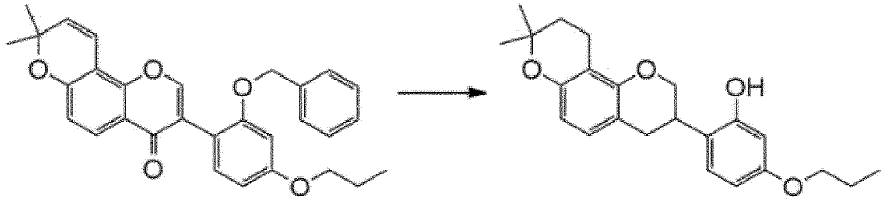

3 g (0.0064 mol) of 3-(2-(benzyloxy)-4-propoxyphenyl)-8,8-dimethyl-pyrano[2,3-f]chromen-4(8H)-one and 0.3 g of Pd/C (50% wet) were added to 60 ml of a mixed solution of ethanol/dichloromethane (1/1) and stirred for 8 hours at 40°C and a hydrogen pressure of 2 atm. The reaction solution was filtered to remove Pd/C, and the filtrate was concentrated under reduced pressure. 31.5 mL of methanol was added to the concentrate which was then dissolved by heating to 60°C. Next, the solution was cooled to 40 to 50°C and 9 mL of purified water was added thereto. Then, the suspension was heated to 62°C, stirred for 1 hour, cooled to 0°C, filtered, and dried to obtain 2.1 g (yield 89.1%) of 2-(8,8-dimethyl-2,3,4,8,9,10-hexahydropyrano[2,3-f]chromen-3-yl)-5-propoxyphenol.

¹H-NMR (CDCl₃) : 6.976(d, 1H, J=8.4Hz), 6.817(d, 1H, J=8.4Hz), 6.452(dd, 1H, J=8.4, 2.0Hz), 6.391(d, 1H, J=8.4Hz), 6.316(d, 1H, J=2.0Hz), 5.600(s, 1H), 4.385(d, 1H, J=10.0Hz), 4.000(t, 1H, J=10.0Hz), 3.812(t, 2H, J=6.4Hz), 3.488(m, 1H), 2.997(dd, 1H, J=15.6, 11.2Hz), 2.837(dd, 1H, J=15.6, 4.4Hz), 2.640(m, 2H), 1.782(t, 2H, J=6.8Hz), 1.765(m, 2H), 1.329(s, 3H), 1.314(s, 3H), 0.994(t, 3H, J=7.2Hz).

¹³C-NMR (CDCl₃) : 159.25, 157.52, 153.05, 152.46, 137.18, 128.81, 128.06, 127.85, 127.66, 127.37, 122.53, 113.15, 109.48, 109.38, 105.59, 100.65, 77.55, 77.43, 77.23, 76.91, 73.83, 70.47, 70.29, 69.81, 32.59, 31.62, 31.07, 27.03, 26.73, 22.80, 17.37, 10.75.

### Example 2: Preparation of 2-(8,8-dimethyl-2,3,4,8,9,10-hexahydropyrano[2,3-f]chromen-3-yl)-5-ethoxyphenol

### 2-1. Synthesis of 3-(2-(benzyloxy)-4-ethoxyphenyl)-8,8-dimethyl-pyrano[2,3-f]chromen-4(8H)-one

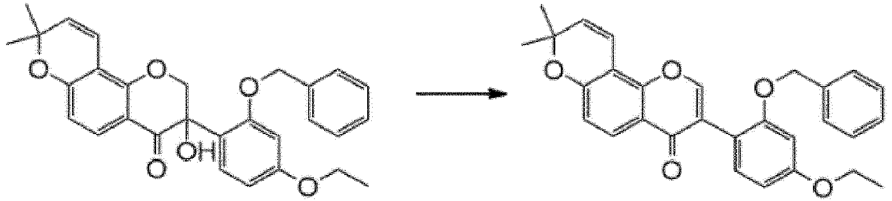

103 g of 95% sulfuric acid was added dropwise to 200 mL of THF, and 10 g (0.0212 mol) of 3-(2-(benzyloxy)-4-ethoxyphenyl)-3-hydroxy-8,8-dimethyl-2,3-dihydropyrano[2,3-f]chromen-4(8H)-one was added to the solution at 10°C or lower. The mixture was warmed to 45°C and stirred for 1 hour. Next, 100 mL of purified water was added to the reaction solution, and then 150 mL of dichloromethane was added thereto. The mixture was stirred and the organic layer was separated. The separated organic layer was washed with a saturated aqueous solution of sodium chloride, dried with anhydrous magnesium sulfate, and then filtered. After concentrating the filtrate under reduced pressure, 33 mL of methanol was added to the concentrate which was then warmed to 50 °C, followed by stirring for 1 hour. Next, the reaction solution was cooled to 25°C, stirred for an additional hour, filtered, and dried to obtain 8.3 g (yield 86.1%) of 3-(2-(benzyloxy)-4-ethoxyphenyl)-8,8-dimethyl-pyrano[2,3-f]chromen-4(8H)-one.

¹H-NMR (CDCl₃) : 8.0651(d, 1H, J=8.72Hz), 7.9207(s, 1H), 7.3550~7.2419(m, 6H), 6.8477(d, 1H, J=8.76Hz), 6.7948(d, 1H, J=10.08Hz), 6.5939(s, 1H), 6.5632(dd, 1H, J=8.32, 2.36Hz), 5.6984(d, 1H, J=10.00Hz), 5.0598 (s, 2H), 4.0347(q, 2H, J=7.00Hz), 1.4949(s, 6H), 1.4090(t, 3H, J=7.00Hz).

¹³C-NMR (CDCl₃) : 176.0099, 160.4870, 157.8353, 157.2428, 153.6205, 152.5834, 137.2505, 132.4447, 130.2857, 128.6290, 127.8392, 127.4578, 127.3635, 126.8988, 122.3177, 118.6943, 115.3109, 115.1300, 114.0880, 109.4124, 105.9419, 101.4197, 77.7783, 77.5471, 77.2300, 76.9119, 70.8369, 63.7899, 28.3316, 15.0114.

### 2-2. Synthesis of 2-(8,8-dimethyl-2,3,4,8,9,10-hexahydropyrano[2,3-f]chromen-3-yl)-5-ethoxyphenol

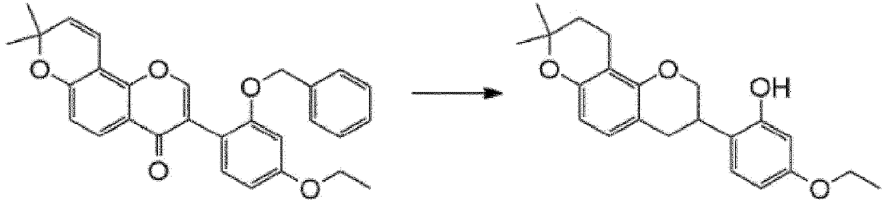

0.5 g (0.0011 mol) of 3-(2-(benzyloxy) -4-ethoxyphenyl)-8,8-dimethyl-pyrano[2,3-f]chromen-4(8H)-one and 0.025 g of Pd/C (50% wet) were added to 20 mL of ethanol and stirred at 0 to 30°C and a hydrogen pressure of 5 atm for 72 hours. The reaction solution was filtered to remove Pd/C, and the filtrate was concentrated under reduced pressure. Methanol was added to the concentrate which was then dissolved by heating to 60 °C. Then, the solution was cooled to 40 to 50°C and purified water was added thereto. The suspension was dissolved by heating, and then cooled to 0 °C, and the precipitated solid was filtered and dried to obtain 2-(8,8-dimethyl-2,3,4,8,9,10-hexahydropyrano[2,3-f]chromen-3-yl)-5-ethoxyphenol.

¹H-NMR (CDCl₃) : 6.989(d, 1H, J=8.4Hz), 6.825(d, 1H, J=8.0Hz), 6.458(dd, 1H, J=8.0, 2.4Hz), 6.387(d, 1H, J=8.4Hz), 6.324(d, 1H, J=2.4Hz), 5.355(s, 1H), 4.386(m, 1H, J=10.4, 3.2, 2.0Hz), 4.007(t, 1H, J=10.4Hz), 3.954(q, 2H, J=7.2Hz), 3.484(m, 1H), 3.006(dd, 1H, J=15.6, 11.2Hz), 2.852(m, 1H, J=15.6, 4.8, 1.6Hz), 2.641(m, 2H), 1.770(t, 2H, J=6.8Hz), 1.378(t, 2H, J=6.8Hz), 1.331(s, 3H), 1.316(s, 3H).

¹³C-NMR (CDCl₃): 158.552, 154.340, 152.719, 152.091, 128.075, 127.465, 119.882, 112.909, 109.305, 109.248, 106.572, 102.504, 73.798, 70.018, 63.450, 32.311, 31.749, 30.614, 26.776, 26.390, 17.116, 14.781.

### Example 3: Preparation of 2-(8,8-dimethyl-2,3,4,8,9,10-hexahydropyrano[2,3-f]chromen-3-yl)-5-propylphenol

### 3-1. Synthesis of 3-(2-(benzyloxy)-4-propylphenyl)-8,8-dimethyl-pyrano[2,3-f]chromen-4(8H)-one

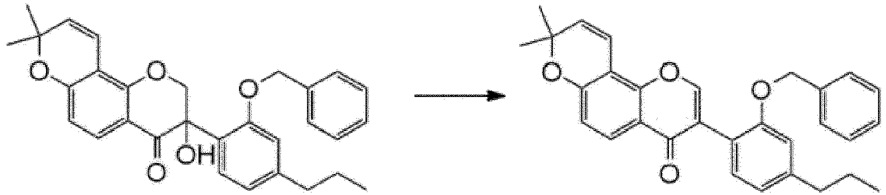

103 g of 95% sulfuric acid was mixed with 150 mL of THF at 0 to 20°C, and a solution of 10 g of 3-(2-(benzyloxy)-4-propylphenyl)-3-hydroxy-8,8-dimethyl-2,3-dihydropyrano[2,3-f]chromen-4 (8H)-one in 50 mL of THF was added to the mixture at 20°C or lower. The resulting mixture was warmed to 40 to 45°C and stirred. After completion of the reaction, the reaction solution was cooled to 20°C or lower, and 100 mL of purified water was added thereto, followed by stirring at 20 to 30°C for 1 hour. The reaction solution was filtered, washed with purified water at room temperature, and dried. The resulting crude solid was separated through column chromatography to obtain 4 g of 3-(2-(benzyloxy)-4-propylphenyl)-8,8-dimethyl-pyrano[2,3-f]chromen-4(8H)-one.

¹H-NMR (CDCl₃): 8.0692 (d, 1H, J=8.75Hz), 7.9285(s, 1H), 7.3560~7.2367(m, 6H), 6.8713~6.7856(m, 4H), 5.6991(d, 1H, J=10.0Hz), 5.0787(s, 2H), 2.5891(t, 2H, J=7.675Hz), 1.6717~1.6265(m, 2H), 1.4955(s, 6H), 0.9530(t, 3H, J=7.325Hz) .

¹³C-NMR (CDCl₃) : 175.6677, 157.0656, 156.5540, 153.4361, 152.3918, 144.6465, 137.3012, 131.4782, 130.1042, 128.3884, 127.5824, 127.2342, 126.7165, 122.4005, 121.1285, 118.8690, 118.5204, 115.1211, 114.9583, 113.5411, 109.2258, 77.5909, 70.7133, 38.2456, 28.1388, 24.4552, 13.9173.

### 3-2. Synthesis of 2-(8,8-dimethyl-2,3,4,8,9,10-hexahydropyrano[2,3-f]chromen-3-yl)-5-propylphenol

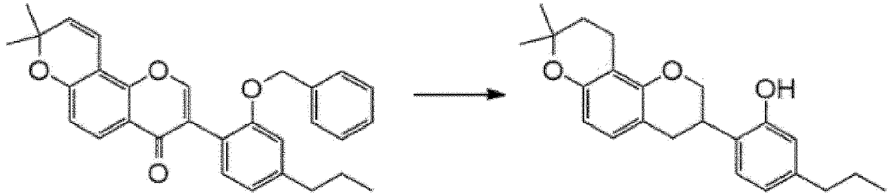

2 g of 3-(2-(benzyloxy)-4-propylphenyl)-8,8-dimethyl-pyrano[2,3-f]chromen-4(8H)-one and 0.2 g of 10% Pd/C (50% wet) were added to 40 ml of ethanol/dichloromethane (1/1) mixture and stirred at 40°C and a hydrogen pressure of 2 atm for 12 hours. The reaction solution was filtered to remove Pd/C, and the filtrate was concentrated under reduced pressure. The concentrate was recrystallized from a n-hexane/dichloromethane solution to obtain 1.3 g of 2-(8,8-dimethyl-2,3,4,8,9,10-hexahydropyrano[2,3-f]chromen-3-yl)-5-propylphenol.

¹H-NMR (CDCl₃) : 7.0347 (d, 1H, J=7.8Hz), 6.8501 (d, 1H, J=8.4Hz), 6.7590(d, 1H, J=7.8Hz), 6.5937(s, 1H), 6.4051(d, 1H, J=8.3Hz), 4.9625(s, 1H), 4.4338(d, 1H, J=10.4Hz), 4.0558(t, 1H, J=10.3Hz), 3.5504(m, 1H), 3.0494(dd, 1H, J=15.5, 11.3Hz), 2.8927(dd, 1H, J=15.6, 4.4Hz), 2.6648(m, 2H), 2.5208(t, 2H, J=7.7Hz), 1.7910(t, 2H, J=6.8), 1.6263(m, 2H), 1.3496(s, 3H), 1.3346(s, 3H), 0.9544(t, 3H, J=7.3Hz).

¹³C-NMR (CDCl₃) : 153.2733, 152.8039, 152.1448, 142.7151, 127.4789, 127.3474, 124.8105, 121.2198, 115.5646, 112.8768, 109.3281, 109.2797, 73.7602, 69.9349, 37.5153, 32.3508, 32.1225, 30.6003, 26.8295, 26.4155, 24.3254, 17.1536, 13.8854.

### Example 4: Preparation of 1-(8,8-dimethyl-2,3,4,8,9,10-hexahydropyrano[2,3-f]chromen-3-yl)-2-(p-tosyloxy)-4-ethoxybenzene

### 4-1. Synthesis of 3-(2-(p-tosyloxy)-4-ethoxyphenyl)-8,8-dimethyl-pyrano[2,3-f]chromen-4(8H)-one

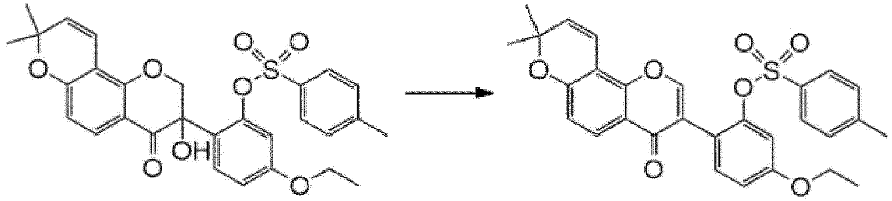

82.5 g of 95% sulfuric acid was mixed with 100 mL of THF at 0 to 20°C, and a solution of 8 g of 3-(2-(p-tosyloxy)-4-ethoxyphenyl)-3-hydroxy-8,8-dimethyl-2,3-dihydropyrano[2,3-f]chromen-4 (8H)-one in 40 ml of THF was added to the mixture at 20°C or lower. The resulting mixture was warmed to 40 to 45°C and stirred. After completion of the reaction, the reaction solution was cooled to 20°C or lower, and 100 mL of purified water was added thereto, followed by stirring at 20 to 30°C for 1 hour. The reaction solution was filtered, washed with 250 mL of purified water at room temperature, and then dried to obtain 5 g of 3-(2-(p-tosyloxy)-4-ethoxyphenyl)-8,8-dimethyl-pyrano[2,3-f]chromen-4(8H)-one.

¹H-NMR (CDCl₃) : 7.854 (d, 1H, J=8.7Hz), 7.7135(s, 1H), 7.4272(d, 2H, J=8.6Hz), 7.3062(d, 1H, J=8.65Hz), 6.9555(d, 1H, J=2.1Hz), 6.8944~6.8625(m, 3H), 6.8349(d, 1H, J=8.7), 6.7729(d, 1H, J=10.0Hz), 5.7603(d, 1H, J=10.0Hz), 4.4109(dd, 2H, J=13.9Hz, 6.95Hz), 2.1531(s, 3H), 1.5308(s, 6H), 1.4310(t, 3H, J=6.9Hz).

¹³C-NMR (CDCl₃): 174.3457, 159.5630, 157.2083, 153.8837, 151.7906, 147.8032, 145.0867, 132.7554, 132.6659, 130.5258, 129.5232, 127.8644, 126.4008, 119.3712, 118.1595, 116.8792, 115.0336, 114.8265, 113.9047, 110.0778, 109.1336, 77.7865, 63.9780, 28.1497, 21.5455, 14.6532.

### 4-2. Synthesis of 1-(8,8-dimethyl-2,3,4,8,9,10-hexahydropyrano[2,3-f]chromen-3-yl)-2-(p-tosyloxy)-4-ethoxybenzene

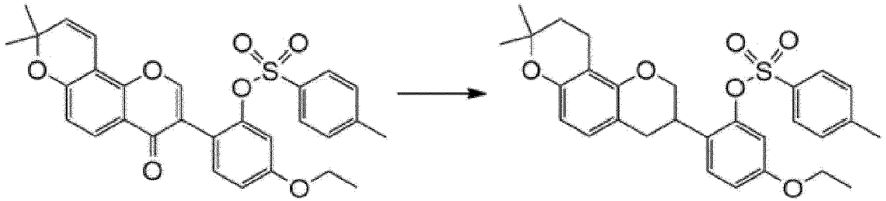

2.5 g of 3-(2-(p-tosyloxy)-4-ethoxyphenyl)-8,8-dimethyl-pyrano[2,3-f]chromen-4 (8H)-one and 0.25 g of 10% Pd/C (50% wet) were added to 50 ml of a ethanol/dichloromethane (1/1) mixture and stirred at 40°C and a hydrogen pressure of 2 atm for 9 hours. The reaction solution was filtered to remove Pd/C, and the filtrate was concentrated under reduced pressure. The concentrate was purified by silica column chromatography and recrystallized from n-hexane/dichloromethane solution to obtain 1.9 g of 1-(8,8-dimethyl-2,3,4,8,9,10-hexahydropyrano[2,3-f]chromen-3-yl)-2-(p-tosyloxy)-4-ethoxybenzene.

¹H-NMR (CDCl₃): 7.7452(d, 2H, J=8.25Hz), 7.2901(d, 2h, J=8.25), 6.9814(d, 1H, J=8.6Hz), 6.791(dd, 1H, J=8.65, 2.45), 6.7515(d, 1H, J=2.5Hz), 6.6930(d, 1H, J=8.35), 6.3636(d, 1H, J=8.35Hz), 4.0019~3.9307(m, 3H), 3.7789(t, 1H, J=10.375), 3.2182(m, 1H), 2.6853(dd, 1H, J=15.6, 11.25Hz), 2.6214(t, 2H, J=6.75Hz), 2.4459(s, 3H), 2.3825(dd, 1H, J=15.6, 3.6), 1.7884(m, 2H), 1.3874(t, 3H, J=6.975Hz), 1.3474(s, 3H), 1.3280(s, 3H).

¹³C-NMR (CDCl₃) : 158.1649, 152.9039, 151.8663, 148.0588, 145.4641, 132.7353, 129.9057, 128.4824, 128.1478, 127.2391, 126.4626, 114.2589, 112.2769, 109.3156, 109.2591, 108.8719, 73.7020, 69.8093, 63.8097, 32.3222, 31.4477, 30.9064, 26.6849, 26.6268, 21.7116, 17.0980, 14.6538.

The above detailed description illustrates and describes the present invention. Additionally, the above description shows and describes only the preferred embodiments of the present invention but, as mentioned above, it is to be understood that the present invention is capable of use in various other combinations, modifications, and environments and is capable of changes or modifications within the scope of the inventive concept as expressed herein, commensurate with the above teachings and/or the skill or knowledge of the art. Accordingly, the above detailed description of the invention is not intended to limit the present invention to the embodiments disclosed herein. Also, it is intended that the appended claims be construed to include alternative embodiments.

## Claims

1. A method for synthesizing a 3-phenyl-2,3,4,8,9,10-hexahydropyrano[2,3-f]chromene derivative, comprising steps of:
dehydrating a compound represented by the following Formula 1 to prepare a compound represented by the following Formula 2; and
reducing the compound represented by Formula 2 to prepare a compound represented by the following Formula 3: 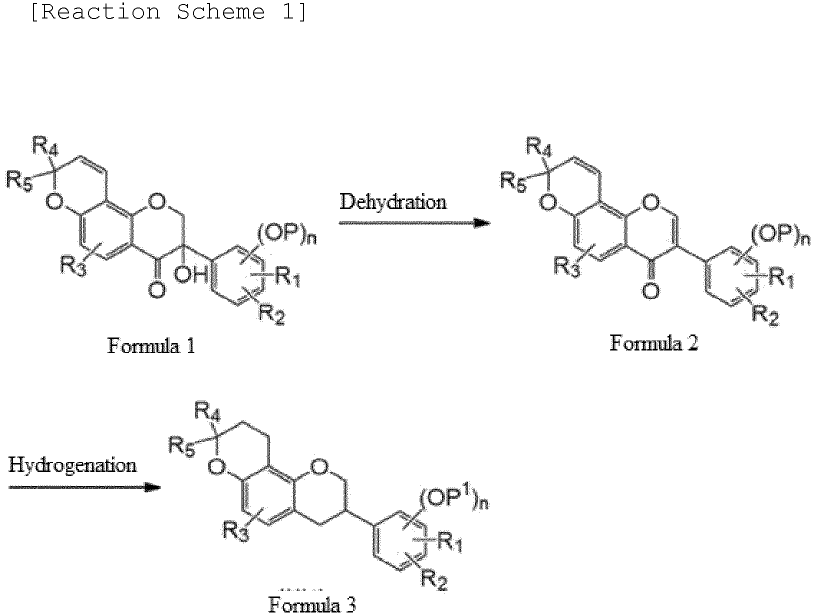 Wherein the Reaction Scheme 1,
R₁ and R₂ are each independently a hydrogen atom; a substituted or unsubstituted, linear or branched C₁-C₆ alkyl group; a halogen atom; a substituted or unsubstituted, linear or branched C₁-C₆ alkoxy group; a substituted or unsubstituted, linear or branched C₁-C₄ thioalkyl group; a substituted or unsubstituted C₆-C₁₂ aryl group; a substituted or unsubstituted allyloxy group; or a substituted or unsubstituted C₆-C₁₂ aryloxy group;
R₃ is a hydrogen atom, a C₁-C₂ alkyl group, a C₁-C₂ alkoxy group, or a halogen atom;
R₄ and R₅ are each independently a hydrogen atom or a C₁-C₆ alkyl group;
P represents a substituted or unsubstituted benzyl group, a substituted or unsubstituted allyl group, methanesulfonyl (MeSO₂), p-toluenesulfonyl (p-TsSO₂), or trimethylphenylsulfonyl;
P¹ is a hydrogen atom, a substituted or unsubstituted allyl group, methanesulfonyl (MeSO₂), p-toluenesulfonyl (p-TsSO₂), or trimethylphenylsulfonyl;
n is 1 to 3;
when OP and OP¹ are plural, P and P¹ are the same or different; and
a substituent of each of the substituted alkyl group, the substituted alkoxy group, the substituted thioalkyl group, the substituted aryl group, the substituted allyloxy group, the substituted aryloxy group, the substituted benzyl group and the substituted allyl group is a benzyloxy group, a halogen atom, a linear or branched C₁-C₅ alkyl group, a linear or branched C₁-C₅ alkoxy group, a linear or branched C₁-C₃ thioalkyl group, or a nitro group.

2. The method of claim 1, wherein
R₁ and R₂ are each independently a hydrogen atom; a substituted or unsubstituted, linear or branched C₁-C₃ alkyl group; a linear or branched C₁-C₃ alkoxy group;
R₃ is a hydrogen atom;
R₄ and R₅ are each independently a hydrogen atom or a C₁-C₃ alkyl group;
P represents a protecting group of a substituted or unsubstituted benzyl group or p-toluenesulfonyl (p-TsSO₂); and
P¹ is a hydrogen atom or p-toluenesulfonyl (p-TsSO₂).

3. The method of claim 1, wherein the Formula 1 is any one compound selected from the group consisting of the following compounds:
3-(2-(benzyloxy)-4-propoxyphenyl)-3-hydroxy-8,8-dimethyl-2,3-dihydropyrano[2,3-f]chromen-4(8H)-one;
3-(2-(benzyloxy)-4-ethoxyphenyl)-3-hydroxy-8,8-dimethyl-2,3-dihydropyrano[2,3-f]chromen-4(8H)-one;
3-(2-(benzyloxy)-4-propylphenyl)-3-hydroxy-8,8-dimethyl-2,3-dihydropyrano[2,3-f]chromen-4(8H)-one; and
3-(2-(p-tosyloxy)-4-ethoxyphenyl)-3-hydroxy-8,8-dimethyl-2,3-dihydropyrano[2,3-f]chromen-4(8H)-one.

4. The method of claim 1, wherein the Formula 3 is any one compound selected from the group consisting of the following compounds:
2-(8,8-dimethyl-2,3,4,8,9,10-hexahydropyrano[2,3-f]chromen-3-yl)-5-propoxyphenol;
2-(8,8-dimethyl-2,3,4,8,9,10-hexahydropyrano[2,3-f]chromen-3-yl)-5-ethoxyphenol;
2-(8,8-dimethyl-2,3,4,8,9,10-hexahydropyrano[2,3-f]chromen-3-yl)-5-propylphenol; and
1-(8,8-dimethyl-2,3,4,8,9,10-hexahydropyrano[2,3-f]chromen-3-yl)-2-(p-tosyloxy)-4-ethoxybenzene.

5. A 3-phenyl-pyrano[2,3-f]chromen-4(8H)-one derivative compound represented by the following Formula 2: 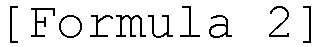 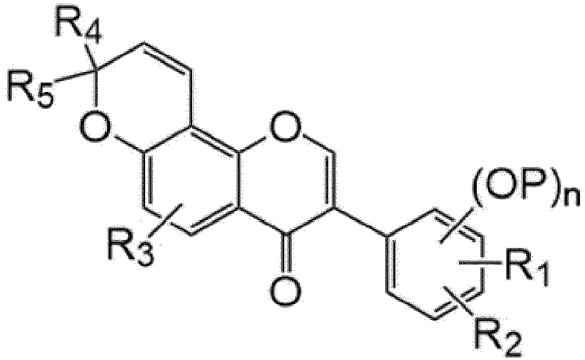 Wherein the Reaction Scheme 2,
R₁ and R₂ are each independently a hydrogen atom; a substituted or unsubstituted, linear or branched C₁-C₆ alkyl group; a halogen atom; a substituted or unsubstituted, linear or branched C₁-C₆ alkoxy group; a substituted or unsubstituted, linear or branched C₁-C₄ thioalkyl group; a substituted or unsubstituted C₆-C₁₂ aryl group; a substituted or unsubstituted allyloxy group; or a substituted or unsubstituted C₆-C₁₂ aryloxy group;
R₃ is a hydrogen atom, a C₁-C₂ alkyl group, a C₁-C₂ alkoxy group, or a halogen atom;
R₄ and R₅ are each independently a hydrogen atom or a C₁-C₆ alkyl group;
P is a substituted or unsubstituted benzyl group, an allyl group, methanesulfonyl (MeSO₂), p-toluenesulfonyl (p-TsSO₂), or trimethylphenylsulfonyl;
n is 1 to 3;
when OP is plural, Ps are the same or different; and
a substituent of each of the substituted alkyl group, the substituted alkoxy group, the substituted thioalkyl group, the substituted aryl group, the substituted allyloxy group, the substituted aryloxy group, the substituted benzyl group and the substituted allyl group is a benzyloxy group, a halogen atom, a linear or branched C₁-C₅ alkyl group, a linear or branched C₁-C₅ alkoxy group, a linear or branched C₁-C₃ thioalkyl group, or a nitro group.
